# EUROPEAN PATENT APPLICATION

(11) **EP 3 476 933 A1**
(43) Date of publication of application: **01.05.2019**
(21) Application number: 17815530.5
(22) Date of filing: 23.06.2017
(51) Int. Cl.: C12N 5/071, C12M 3/00, A61L 27/60

(54) **THREE-DIMENSIONALLY CULTURED SKIN SHEET, CELL CULTURING VESSEL USED FOR PRODUCTION THEREOF, AND METHOD FOR PRODUCING THREE-DIMENSIONALLY CULTURED SKIN SHEET**

(30) Priority: 24.06.2016 JP 2016125843; 28.12.2016 JP 2016256778
(71) Applicant: Shiseido Company, Ltd., Tokyo 104-0061 (JP)
(72) Inventor: DENDA, Mitsuhiro, Yokohama-shi Kanagawa 224-8558 (JP); DENDA, Sumiko, Yokohama-shi Kanagawa 224-8558 (JP); KUMAMOTO, Junichi, Sapporo-shi Hokkaido 060-0812 (JP); KOBAYASHI, Yasuaki, Sapporo-shi Hokkaido 060-0812 (JP); NAGAYAMA, Masaharu, Sapporo-shi Hokkaido 060-0812 (JP)
(74) Representative: Liesegang, Eva
(86) International application number: PCT/JP2017/023302
(87) International publication number: WO 2017/222065

(57) **Abstract**

A thick three-dimensional culture skin sheet is provided. The invention provides a three-dimensional culture skin sheet comprising at least epidermal cells and having concavoconvexities in at least one portion of the base section. The invention further provides a cell culturing vessel to be used for production of the three-dimensional culture skin sheet, having on at least one portion of the culture surface a porous film comprising convex sections for formation of concavoconvexities in the base section of the three-dimensional culture skin sheet. The invention still further provides a method for preparing a three-dimensional culture skin sheet using the cell culturing vessel.

## Description

### FIELD

The present invention relates to a three-dimensional culture skin sheet. The invention further relates to a cell culturing vessel for use in production of a three-dimensional culture skin sheet. The invention still further relates to a method for preparing a three-dimensional culture skin sheet. The present application has been filed claiming the priority of a Japanese application filed on June 24, 2016 (Japanese Patent Application No. 2016-125843) and a Japanese application filed on December 28, 2016 (Japanese Patent Application No. 2016-256778).

### BACKGROUND

The skin is an organ that covers the surface of the body, separating the interior of the body from its exterior. Skin functions as a physical barrier and protects the interior of the body from dryness and infiltration of hazardous substances, thus performing an indispensable role for life maintenance.

The skin of higher vertebrates primarily consists of the epidermis, dermis and subcutaneous tissue layers, in order from the outermost layer. The interface between the epidermis and the dermis is corrugated, and the corrugations are known to flatten with age (see NPL 1, for example). The epidermis is composed mainly of cells known as keratinocytes, the keratinocytes dividing at the deepest part (basal lamina) of the epidermis while migrating to the surface and differentiating toward the upper layer into the stratum spinosum, granular layer and stratum corneum, eventually being shed as old skin. This cycle takes about 4 weeks in humans.

It has been attempted to construct various *ex vivo* cultured skin models that simulate epidermal structure and its function, and some of them are already commercially available (see PTL 1, NPL 2 and NPL 3, for example). It has also been attempted to cast a collagen gel into a mold having grooves formed by photolithography and to seed keratinocytes on a scaffold formed by seeding fibroblasts into the collagen gel, thus creating a three-dimensional culturing skin model (PTL 2, NPL 4), and additionally, it has been attempted to use cell culture container materials that have undulations on the culture surface, in order to examine their effect on the arrangement of stem cells present in primary human keratinocytes (NPL 5), while cell culture container material surfaces made of the biodegradable polymer chitosan, polycaprolactone or mixtures thereof, have also been used to examine the effects on differentiation of human keratinocytes (NPL 6). It has further been attempted to prepare three-dimensionally cultured skin by seeding keratinocytes on a human decellularized dermal matrix obtained by removing the epithelial tissue and cell components from skin tissue harvested from human donated samples (NPL 7).

Such cultured skin models are used for drug safety testing or basic research for cosmetic products or skin-applied drugs, and they are garnering increasing interest as animal-substituting models, but a cultured skin model having the same level of thickness and barrier function as human skin has not yet been obtained.

### [CITATION LIST]

### [PATENT LITERATURE]

[PTL 1] Japanese Unexamined Patent Publication No. 2012-235921
[PTL 2] U.S. Patent Application Publication No. 2011/0171180

### [NON PATENT LITERATURE]

[NPL 1] Lavker R.M. Structural alterations in exposed and unexposed aged skin. J Invest Dermatol. 1979 Jul; 73(1):59-66.
[NPL 2] Bell E., et al., The reconstitution of living skin. J Invest Dermatol. 1983 Jul; 81(1 Suppl):2s-10s.
[NPL 3] "Keratinocyte three-dimensional culture starter kit" [online], May 11, 2009, Funakoshi Corp., [searched on June 16, 2016], internet <URL:http://www.funakoshi.cojp/contents/3571>
[NPL 4] Clement AL, Moutinho TJ Jr, Pins GD. Micropatterned dermal-epidermal regeneration matrices create functional niches that enhance epidermal morphogenesis. Acta Biomater. 2013 Dec; 9(12):9474-84.
[NPL 5] Viswanathan P., et al., Mimicking the topography of the epidermal-dermal interface with elastomer substrates. Integr Biol (Camb). 2016 Jan; 8 (1):21-9.
[NPL 6] Salerno S., Polymeric membranes modulate human keratinocyte differentiation in specific epidermal layers. Colloids Surf B Biointerfaces. 2016 Oct 1; 146:352-62.
[NPL 7] Kato H., et al., Fabrication of Large Size Ex Vivo-Produced Oral Mucosal Equivalents for Clinical Application. Tissue Eng Part C Methods. 2015 Sep; 21(9):872-80.

### SUMMARY

### [TECHNICAL PROBLEM]

While cultured skin models have been developed as animal-substituting models, the existing models that have been constructed *in vitro* still cannot be said to have sufficient thickness compared to living epidermis. The existing cultured skin models have also failed to exhibit a sufficient barrier function compared to skin obtained from animals, and particularly humans. Moreover, when using the previously marketed three-dimensional culture kits, one problem that has been faced is that three-dimensional cultured skin with the desired thickness can only be obtained when primary cultured epidermal cells with no subculturing or with a low passage number (for example, a passage number of 0 or 1) have been used. For this reason, it has been a goal to devise a technology that allows three-dimensional cultured skin having equal or nearly equal thickness to that of live skin, to be obtained in an economical, stable and convenient manner not only when primary cultured cells with a low passage number are used, but even when any constituent cells of the skin are used.

### [SOLUTION TO PROBLEM]

As a result of much research conducted by the present inventors in light of the need described above, it has been found that by culturing epidermal cells on a porous film having convex sections on the culture surface, it is possible to obtain a three-dimensional culture skin sheet having greater thickness than by prior art culturing methods, and the present invention has thus been devised.

Specifically, the present invention encompasses the following inventions.
[1] A three-dimensional culture skin sheet comprising at least epidermal cells and having concavoconvexities in at least one portion of the base section.
[2] The three-dimensional culture skin sheet according to [1], wherein the epidermal cells are keratinocytes.
[3] The three-dimensional culture skin sheet according to [1] or [2], which further includes an epidermal cell-free region in at least one portion of the interior of the three-dimensional culture skin sheet.
[4] The three-dimensional culture skin sheet according to any one of [1] to [3], wherein the average thickness of the three-dimensional culture skin sheet is 20 µm or greater.
[5] The three-dimensional culture skin sheet according to any one of [1] to [4], wherein the height of the concavoconvexities is 1 µm to 300 µm on average.
[6] The three-dimensional culture skin sheet according to any one of [1] to [4], wherein the height of the concavoconvexities is 1 µm to 90 µm on average.
[7] The three-dimensional culture skin sheet according to any one of [1] to [6], wherein the separation width between adjacent concavoconvexities is 1 µm to 300 µm on average.
[8] The three-dimensional culture skin sheet according to any one of [1] to [6], wherein the separation width between the concavoconvexities is 1 µm to 100 µm on average.
[9] The three-dimensional culture skin sheet according to any one of [1] to [8], which further comprises a porous film having convex sections that are in contact with the base section.
[10] The three-dimensional culture skin sheet according to [9], wherein the convex sections are made of a non-biological substance.
[11] A cell culturing vessel to be used for production of a three-dimensional culture skin sheet according to any one of [1] to [10], having a porous film provided on at least one portion of the culture surface and convex sections arranged on the porous film and serving to form concavoconvexities on the base section of the three-dimensional culture skin sheet.
[12] The cell culturing vessel according to [11], comprising a non-biological substance.
[13] The cell culturing vessel according to [11] or [12], wherein the height of the convex sections is 1 µm to 300 µm on average.
[14] The cell culturing vessel according to [11] or [12], wherein the height of the convex sections is 1 µm to 90 µm on average.
[15] The cell culturing vessel according to any one of [11] to [14], wherein the separation width between adjacent convex sections is 1 µm to 300 µm on average.
[16] The cell culturing vessel according to any one of [11] to [14], wherein the separation width between adjacent convex sections is 1 µm to 100 µm on average.
[17] The cell culturing vessel according to any one of [11] to [16], wherein the convex sections comprise bead shapes, rectangular columnar shapes, pyramidal shapes, frustum shapes, bell shapes and/or lattice shapes.
[18] A method for preparing a three-dimensional culture skin sheet, comprising (1) a step of seeding cells that include epidermal cells suspended in a culture medium, in a cell culturing vessel having a porous film provided on at least one portion of the culture surface and convex sections arranged on the porous film and serving to form concavoconvexities on the base section of the three-dimensional culture skin sheet, and (2) a step of contacting culture medium with the outer side of the porous film of the cell culturing vessel and carrying out culturing.
[19] The method according to [18], further comprising (3) a step of removing the culture medium in the cell culturing vessel, exposing the skin cells to the air and culturing them.
[20] The method according to [18] or [19], wherein the convex sections are made of a non-biological substance.
[21] The method according to any one of [18] to [20], wherein the height of the convex sections is 1 µm to 300 µm on average.
[22] The method according to any one of [18] to [20], wherein the height of the convex sections is 1 µm to 90 µm on average.
[23] The method according to any one of [18] to [22], wherein the separation width between adjacent convex sections is 1 µm to 300 µm on average.
[24] The method according to any one of [18] to [22], wherein the separation width between adjacent convex sections is 1 µm to 100 µm on average.
[25] The method according to any one of [18] to [24], wherein the convex sections comprise bead shapes, rectangular columnar shapes, pyramidal shapes, frustum shapes, bell shapes and/or lattice shapes.
[26] The method according to any one of [18] to [25], wherein the epidermal cells include epidermal cells with a passage number of 2 or greater after isolated from biological tissue and then cultured.
[27] The method according to any one of [18] to [26], wherein the epidermal cells are keratinocytes.

### [ADVANTAGEOUS EFFECTS OF INVENTION]

With the three-dimensional culture skin sheet of the invention, it is possible to obtain a base section (epidermal basal membrane-like structure) having concavoconvexities that have not been present in three-dimensional culture skin sheets of the prior art, as well as a thickened three-dimensional culture skin sheet. It is thereby possible to provide a three-dimensional skin model that is useful for safety testing and basic research for cosmetics or skin-applied drugs. Furthermore, using the cell culturing vessel of the invention, it is possible to provide, in a stable and economical manner, a thickened three-dimensional culture skin sheet which has not been obtainable in the prior art. Using the culturing method of the invention, as well, it is possible to provide, in a stable and economical manner, a thickened three-dimensional culture skin sheet which has not been obtainable in the prior art.

### BRIEF DESCRIPTION OF DRAWINGS

FIG. 1 is a conceptual drawing of the present invention. In the drawing, (a) shows a cross-sectional view of a cell culturing vessel of the invention. Drawing (b) shows a magnified view of a portion of drawing (a).
FIG. 2 is a conceptual drawing showing the structure of a three-dimensional culture skin sheet of the invention.
FIG. 3 is a conceptual drawing of one aspect of the invention.
FIG. 4 is a conceptual drawing of one aspect of the invention.
FIG. 5 is a conceptual drawing of one aspect of the invention.
FIG. 6 is a conceptual drawing of one aspect of the invention.
FIG. 7 is a conceptual drawing of one aspect of the invention.

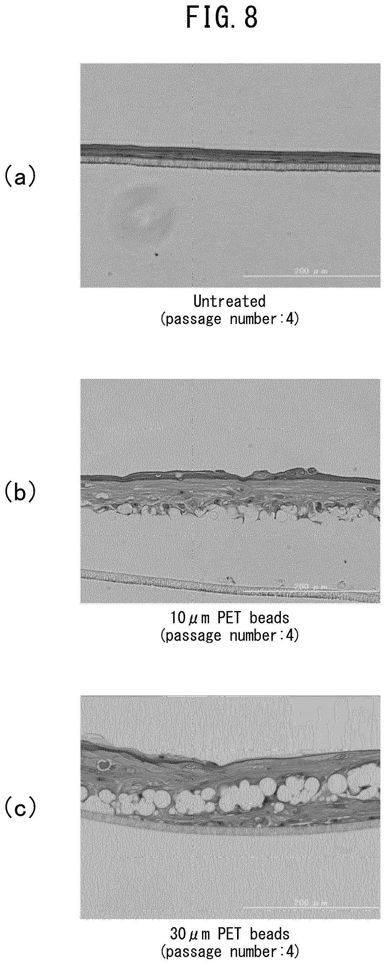

FIG. 8 is a photograph showing a cross-sectional view of a three-dimensional culture skin sheet having concavoconvexities formed in the base section using beads. Drawing (a) is a photograph of a three-dimensional culture skin sheet constructed on a culture surface without beads. Drawing (b) is a photograph of a three-dimensional culture skin sheet constructed on a culture surface with PET beads having a mean particle diameter of 10 µm. Drawing (c) is a photograph of a three-dimensional culture skin sheet constructed on a culture surface with PET beads having a mean particle diameter of 30 µm. For (a) to (c), human keratinocytes with a passage number of 4 were used.

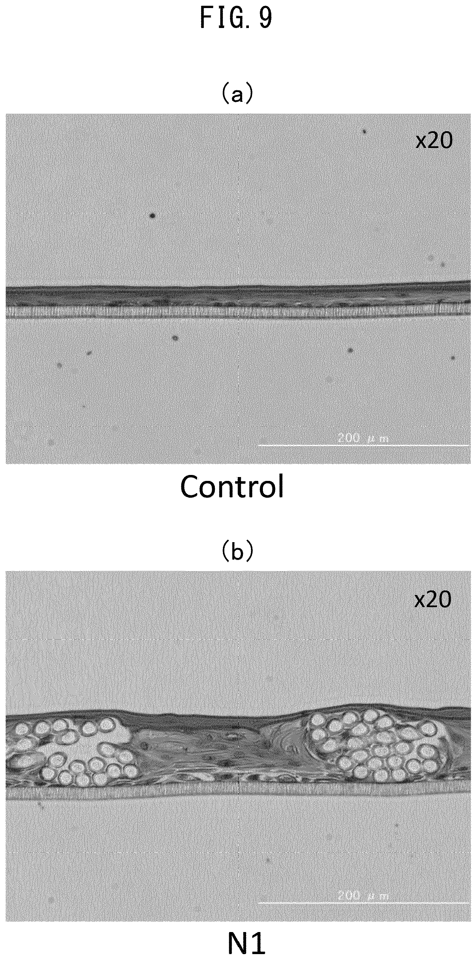

FIG. 9 is a photograph showing a cross-sectional view of a three-dimensional culture skin sheet having concavoconvexities formed in the base section by fibers. Drawing (a) is a photograph of a three-dimensional culture skin sheet constructed on a culture surface without polyester fibers (control). Drawing (b) is a photograph of a three-dimensional culture skin sheet constructed on a culture surface having polyester fibers (a mesh form having average spacings of 50 µm weft and 50 µm warp, and an average thickness of 50 µm, hereunder referred to as "N1"). For (a) and (b), human keratinocytes with a passage number of 4 were used.
FIG. 10 shows magnified views of polyester fibers used in the Examples for formation of concavoconvexities in a three-dimensional culture skin sheet of the invention.
FIG. 11A is a cross-sectional view of a three-dimensional culture skin sheet having concavoconvexities formed in the base section using a cell culturing vessel having the different fibers shown in FIG. 10. Each was observed at a magnification of 20x.
FIG. 11B shows cross-sections of three-dimensional culture skin sheets having concavoconvexities formed in the base section using cell culturing vessels having the different fibers shown in FIG. 10. Each was observed at a magnification of 20x.

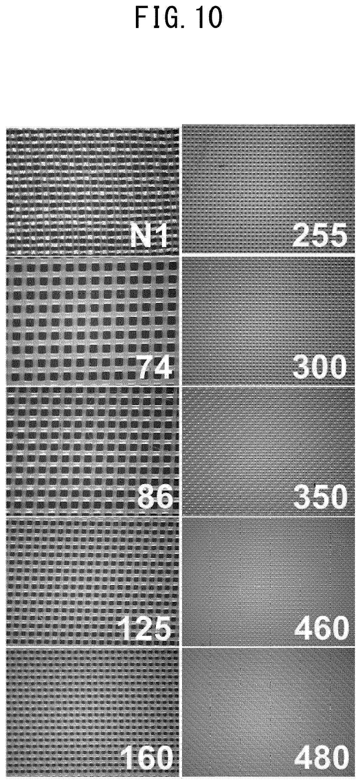

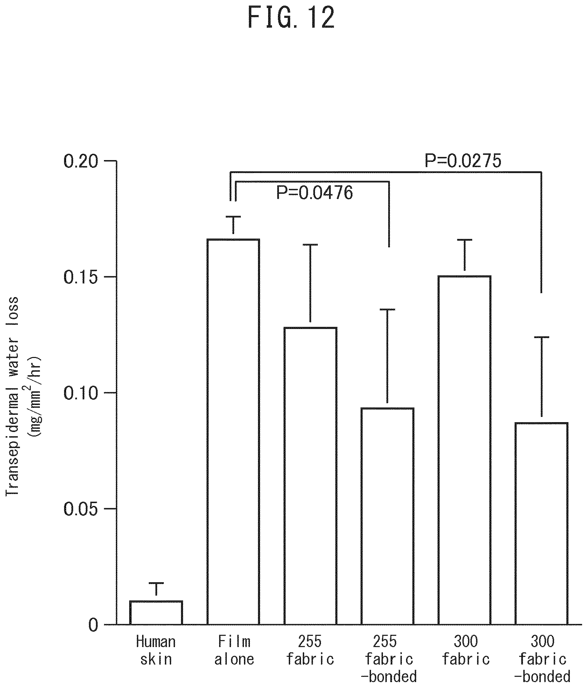

FIG. 12 is a graph for evaluation of the barrier function (moisture transpiration) of three-dimensional culture skin sheets prepared using cell culturing vessels having the fibers shown in FIG. 10. The moisture transpiration was examined for human skin, a skin sheet cultured on a porous film without concavoconvexities (film alone) a skin sheet prepared using a culture vessel having the No. 225 fabric placed on a porous film (No. 225 fabric), a skin sheet prepared using a culture vessel having the No. 225 fabric placed on a porous film (No. 225 fabric-bonded), a skin sheet prepared using a culture vessel having the No. 300 fabric placed on a porous film (No. 300 fabric), and a skin sheet prepared using a culture vessel having the No. 300 fabric placed on a porous film (No. 300 fabric-bonded).

### DESCRIPTION OF EMBODIMENTS

Embodiments for carrying out the invention will be described in detail below with reference to the accompanying drawings, but the technical scope of the invention is not limited only to these embodiments.

### <Three-dimensional culture skin sheet construction example>

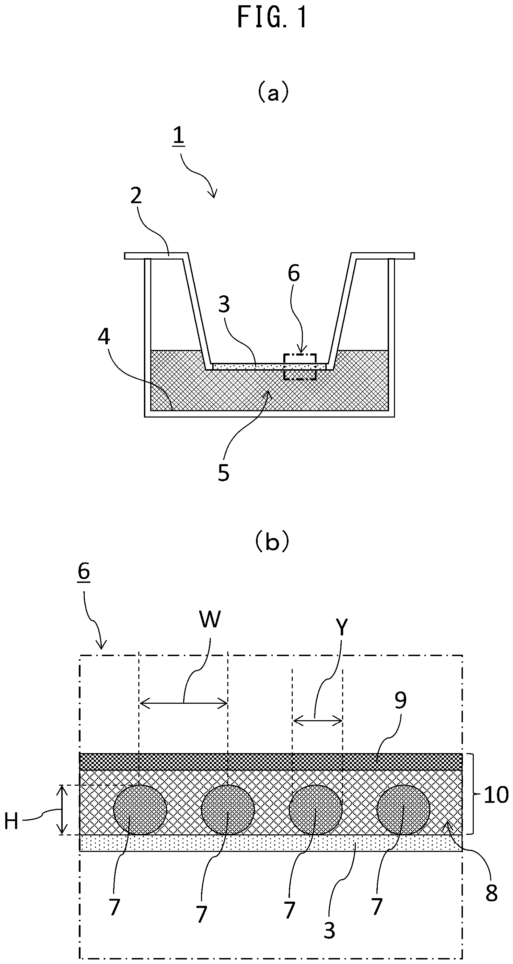

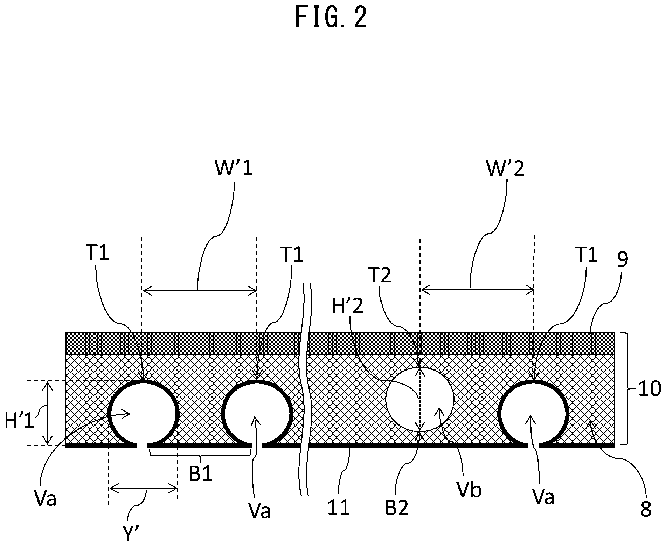

Fig. 1 is a cross-sectional view showing a cell culturing vessel 1 to be used for this embodiment, and a cross-sectional magnified view 6 of a portion of the culture surface of the same. The cell culturing vessel 1 includes a cell culture insert 2 in which the cells are seeded, and a bottom well 4 for filling with culture medium around the outside of the cell culture insert 2. The cell culture insert 2 comprises a porous film 3. The nutrients and oxygen can be supplied from the culture medium 5 filling the outside of the cell culture insert 2 through the porous film 3 to the cells on the porous film 3. For this embodiment of the invention, the surface of the porous film 3 comprises convex sections 7 for formation of concavoconvexities on the three-dimensional culture skin sheet 10 of the invention. By seeding and culturing epidermal cells in the cell culturing vessel 1 of the invention, the epidermal cells become overlaid, and it is possible to obtain a three-dimensional culture skin sheet 10 having concavoconvexities in at least one portion of the base section 11 of the three-dimensional culture skin sheet 10 (the bold lines in Fig. 2), and/or an epidermal cell-free region in at least one portion of the interior of the three-dimensional culture skin sheet.

According to the invention, the "base section" of the three-dimensional culture skin sheet 10 is the side of the epidermal cells formed as a sheet that contacts with the surface of the cell culturing vessel 1 when the epidermal cells have been seeded in the cell culturing vessel, and for example, it is the side in contact with the surface of the porous film 3 shown in Fig. 1 and/or with the convex sections 7 (11 and the bold lines in Fig. 2). Also according to the invention, while the "base section" is a structure corresponding to the basal membrane formed between the epidermis and dermis in living skin tissue, it does not necessarily need to have the same membrane structure as a living basal membrane. That is, the "base section" for the invention includes a "epidermal basal membrane-like structure" having a concavoconvex shape in at least one portion, the epidermal basal membrane-like structure including epidermal cells. The epidermal basal membrane-like structure may also include a basal membrane as formed by epidermal cells in living skin tissue.

Fig. 2 is a cross-sectional view of a three-dimensional culture skin sheet 10 having the porous film 3 and convex sections 7 of Fig. 1(b) removed. The three-dimensional culture skin sheet 10 includes a stratum corneum 9 which lacks cell nuclei, and epidermal cells 8 in addition to the stratum corneum 9. Due to the convex sections 7 present on the porous film 3, the three-dimensional culture skin sheet 10 has concavoconvexities formed in a manner with cells surrounding epidermal cell-free regions Va and/or epidermal cell-free regions Vb in the epidermal cell-free region inside the three-dimensional culture skin sheet.

According to the invention, "concavoconvexities" in the base section 11 of the three-dimensional culture skin sheet 10 are formed in at least one portion of the side of the three-dimensional culture skin sheet of the invention that is in contact with the culture surface of the cell culturing vessel, and the term refers to undulations that are larger than undulations accidentally obtained when epidermal cells have been cultured on the surface of a flat culturing instrument. According to the invention, the convexities of the concavoconvexities of the three-dimensional culture skin sheet 10 are portions of the base section 11 that protrude toward the direction of air exposure (the upper layer) of the cell culturing vessel, and for example, they are the convex sections T1 of the three-dimensional culture skin sheet in Fig. 2, and the sections formed by the convex sections 7 on the porous film 3 in Fig. 1. Also according to the invention, the concavities of the concavoconvexities of the three-dimensional culture skin sheet are the sections that are depressed in the direction of the base section of the epidermis, opposite from the convexities, and for example, they are the recesses B1 of the three-dimensional cell sheet in Fig. 2. The distance between the tip of any convex section T1 of the three-dimensional culture skin sheet and the maximum depression of a recess B1 of the three-dimensional culture skin sheet adjacent to that tip, in the direction perpendicular to the culture surface, may be considered to be the height H'1 of the concavoconvexities of the three-dimensional culture skin sheet. The form and height H'1 of the concavoconvexities formed in the three-dimensional culture skin sheet 10 depend on the form and height H of the convex sections 7 on the porous film 3. Also, the separation width W'1 between convex sections of the three-dimensional culture skin sheet formed in the three-dimensional culture skin sheet 10 depends on the separation width W between the convex sections formed by the convex sections 7 on the porous film 3. Moreover, the width Y' of the convex sections of the three-dimensional culture skin sheet formed in the three-dimensional culture skin sheet 10 depends on the width Y of the convex sections formed by the convex sections 7 on the porous film 3.

The height H'1 of the concavoconvexities of the three-dimensional culture skin sheet, the separation width W'1 between the convex sections of the three-dimensional culture skin sheet and/or the width Y' of the convex sections of the three-dimensional culture skin sheet may be measured, for example, using a commercially available optical microscope with preparation of an HE-stained tissue section, or they may be measured by observation using a fluorescent microscope, confocal microscope or two-photon laser microscope, with immunohistochemical staining, or they may be measured by observation using an electron microscope, without any particular restrictions. The height H'1 of the concavoconvexities of the three-dimensional culture skin sheet can be measured from an image taken using a camera-equipped apparatus such as a common microscope or fluorescent microscope. The microscope used may be, for example, a BX51 system industrial (transmission/reflection) microscope by Olympus Corp. The camera used may be, for example, a DP71 microscope digital camera by Olympus Corp. The taken image may be loaded into computer analysis software and analyzed by an established image analysis method.

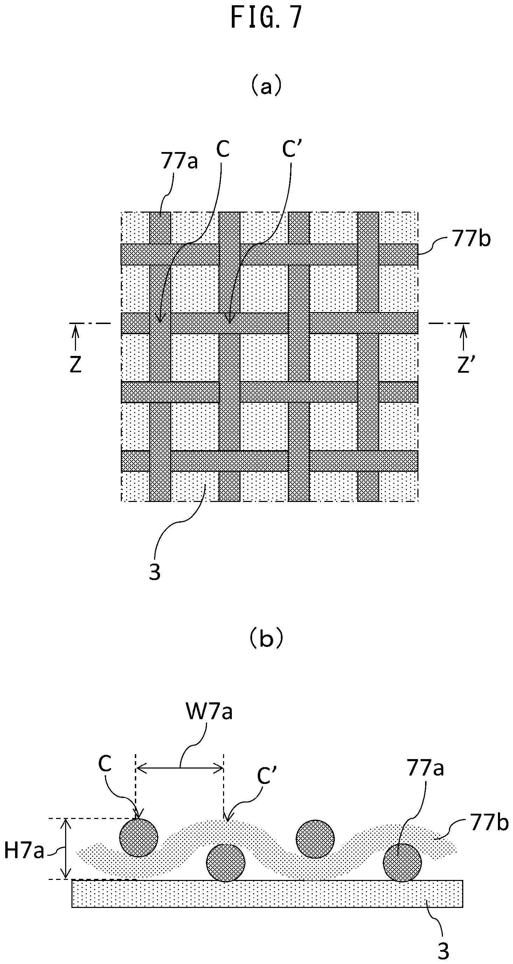

According to the invention, a portion of any cross-section of the three-dimensional culture skin sheet 10 will sometimes have an epidermal cell-free region Vb where no concavoconvexities are formed in the base section 11 of the three-dimensional culture skin sheet 10, as shown at right in Fig. 2. Since cells are present in the lower region of the bottom section B2 of the epidermal cell-free region of the epidermal cell-free region Vb of the cross-section, when viewing the cross-section it may appear not to form a concavoconvex shape connected with the outside of the three-dimensional culture skin sheet 10. For example, when the convex sections 7 on the porous film 3 are bead shapes, an epidermal cell-free region Vb may be observed in a cross-section where the convex sections 7 are not in contact with the porous film 3. Or when the convex sections 7 on the porous film 3 consist of a fiber-woven mesh form (see Fig. 7 described below, for example), an epidermal cell-free region Vb may be observed in a cross-section including a location where fibers are not bonded to the porous film 3. An epidermal cell-free region Vb may also be observed when the convex sections 7 are free from the top of the porous film 3 in the cell culture and cells infiltrate and engraft onto the bottom parts of the convex sections 7. According to the invention, the concavoconvexities of the three-dimensional culture skin sheet 10 include shapes where the epidermal cells 8 surround epidermal cell-free regions Vb that include the examples mentioned above. In most cases the height H'2 of the epidermal cell-free region Vb approximately matches the height H'1 of the concavoconvexities of the three-dimensional culture skin sheet.

Throughout the present specification, the term three-dimensional" means that the cells are stacked in the vertical direction to form an overlaid state, which differs from an approximately single cell layer obtained by culturing conventional adherent cells in a cell culture dish or the like, i.e. a two-dimensional cell layer form. For example, the three-dimensional culture skin sheet of the invention has concavoconvexities in an epidermal basal membrane-like structure, as a three-dimensionalized structure with thickness, in which the epidermal cells are overlaid.

Throughout the present specification, the term "three-dimensional culture skin sheet" is distinguished from skin tissue inherently found in living bodies, and refers to three-dimensional skin-like tissue obtained by seeding a cell group including cells from skin tissue obtained by degrading intercellular adhesion proteins to separate them, and/or skin-forming cells obtained by inducing differentiation from pluripotent stem cells, in a cell culturing vessel and culturing them on the cell culturing vessel, to reconstitute the cells into a sheet. According to the invention, the cells forming the three-dimensional culture skin sheet include epidermal cells, and they may be considered to be a three-dimensional culture epidermal sheet. According to the invention, the three-dimensional culture skin sheet or three-dimensional culture epidermal sheet may also include cells other than epidermal cells, such as cells other than epidermal cells that are components of the epidermis (such as melanocytes, Langerhans cells and Merkel cells), and/or cells present in dermal tissue (fibroblasts, neurons, mast cells, plasmocytes, vascular endothelial cells, histiocytes and Meissner corpuscles). The three-dimensional culture skin sheet of the invention may also be one comprising a porous film 3 having convex sections in contact with the base section 11.

The cells composing the three-dimensional culture skin sheet of the invention may be derived from any animal, but they are preferably derived from a vertebrate, more preferably from a mammal, and most preferably from a human.

According to the invention, the term "epidermal cells" means cells including all cells composing the epidermis at different stages of differentiation. Epidermal cells mainly consist of cells known as keratinocytes. In living bodies, epidermal tissue is formed of epidermal cells at different stages of differentiation that are overlaid in a laminar fashion. The deepest portion of the epidermis is called the basal lamina or basal cell layer (hereunder referred to as "basal lamina"), where cylindrical cells form a single layer and are thought to include stem cells. The basal lamina is also an interface with the dermis, and the stratum spinosum is present on its upper layer. In living bodies, a papillary layer forming the dermis is present directly under the basal lamina.

The stratum spinosum is also known as the squamous cell layer, and is composed of approximately 2 to 10 layers in biological tissue. The layer is called the "stratum spinosum" because interconnected "spines" are observed. Only cells of the basal lamina have proliferation potency, and with progressive stages of differentiation, epidermal cells migrate to the outer layer while changing to a flat form.

When the differentiation stage progresses from the stratum spinosum, a granular layer with keratohyalin granules and lamellar granules (granulocyte layer) is formed. The granular layer is composed of about 2 or 3 layers in biological tissue. When the differentiation stage progresses even beyond the granular layer, the cell nuclei are lost and the stratum corneum (stratum corneum cell layer) is formed. The epidermis is largely composed of these 4 types of layers, but epidermal tissue also includes melanocytes, Langerhans cells and Merkel cells in addition to epidermal cells, each of which prevent ultraviolet rays from reaching the dermis, perform an important role in skin immunological function, and contribute to sensation. According to the invention, the three-dimensional culture skin sheet may contain cells other than epidermal cells, and it may be modified as appropriate according to the purpose of use.

The "barrier function" of skin generally refers to the function of preventing loss of moisture or biological components from the body, or the function of preventing entrance of foreign matter (microorganisms, viruses and dust) into the body from the exterior. Throughout the present specification, the barrier function of the three-dimensional culture skin sheet of the invention can be evaluated by examining the transepidermal water loss (TEWL). Lower transepidermal water loss, or moisture permeation, from the three-dimensional culture skin sheet represents a higher barrier function. According to the invention, the method used to examine the transepidermal water loss may be a method commonly employed by those skilled in the art (see Kumamoto J., Tsutsumi M., Goto M., Nagayama M., Denda M., Japanese Cedar (Cryptomeria japonica) pollen allergen induces elevation of intracellular calcium in human keratinocytes and impairs epidermal barrier function of human skin ex vivo. Arch. Dermatol. Res. 308:49-54, 2016, for example).

According to the invention, the average lower limit for the height H'1 of the concavoconvexities of the three-dimensional culture skin sheet may be 1 µm or greater, 5 µm or greater, 10 µm or greater, 15 µm or greater, 20 µm or greater, 25 µm or greater or 30 µm or greater, for example. According to the invention, the average upper limit for the height H'1 of the concavoconvexities of the three-dimensional culture skin sheet may be 300 µm or smaller, 250 µm or smaller, 200 µm or smaller, 150 µm or smaller, 100 µm or smaller, 80 µm or smaller or 75 µm or smaller. According to the invention, the average for the height H'1 of the concavoconvexities of the three-dimensional culture skin sheet may be 1 µm to 300 µm, 1 µm to 200 µm, 1 µm to 100 µm, 5 µm to 250 µm, 10 µm to 200 µm, 15 µm to 150 µm, 20 µm to 100 µm, 25 µm to 80 µm or 30 µm to 75 µm, for example.

According to the invention, the average lower limit for the separation width W'1 (or W'2) between the convex sections of the three-dimensional culture skin sheet of the may be 1 µm or greater, 5 µm or greater, 8 µm or greater, 10 µm or greater, 12 µm or greater, or 15 µm or greater, for example. The average upper limit for the separation width W'1 (or W'2) between the convex sections of the three-dimensional culture skin sheet may be 300 µm or smaller, 250 µm or smaller, 200 µm or smaller, 180 µm or smaller, 170 µm or smaller, 150 µm or smaller, 125 µm or smaller, 100 µm or smaller or 90 µ or smaller, for example. According to the invention, the average for the separation width W'1 (or W'2) between the convex sections of the three-dimensional culture skin sheet may be 1 µm to 300 µm, 1 µm to 200 µm, 1 µm to 100 µm, 1 µm to 90 µm, 5 µm to 250 µm, 8 µm to 200 µm, 10 µm to 180 µm, 12 µm to 100 µm, 25 µm to 80 µm or 30 µm to 75 µm, for example.

According to the invention, the average lower limit for the width Y'1 of the convex sections of the three-dimensional culture skin sheet may be 1 µm or greater, 5 µm or greater, 10 µm or greater, 15 µm or greater, 20 µm or greater or 25 µm or greater, for example. According to the invention, the average upper limit for the width Y'1 of the convex sections of the three-dimensional culture skin sheet may be 300 µm or smaller, 250 µm or smaller, 200 µm or smaller, 150 µm or smaller, 100 µm or smaller, 80 µm or smaller or 75 µm or smaller, for example. According to the invention, the average for the width Y'1 of the convex sections of the three-dimensional culture skin sheet may be 1 µm to 300 µm, 5 µm to 250 µm, 10 µm to 200 µm, 15 µm to 150 µm, 20 µm to 100 µm, 25 µm to 80 µm or 30 µm to 75 µm, for example.

If at least one portion of the three-dimensional culture skin sheet has concavoconvexities in the base section 11, a three-dimensional culture skin sheet with thicker epidermal cell layers on the base section 11 (for example, the stratum spinosum, granular layer and/or horny layer) will be obtained, compared to one without concavoconvexities. The three-dimensional culture skin sheet obtained by the invention has an excellent effect of thickening simply by physical formation of concavoconvexities in the base section 11 by convex sections formed of non-biological substances, instead of relying on convex sections composed of cells and biological substances such as cell adhesion proteins. In addition, when the convex sections are convex sections composed of non-biological substances, it is possible to stably maintain the concavoconvex structure on the base section of the three-dimensional culture skin sheet without degradation of the convex sections, whether used during the culturing period or afterwards, and to thus continuously maintain the effect of the invention. The average height, shapes and separation width of the concavoconvexities of the three-dimensional culture skin sheet of the invention can be controlled by the heights, shapes and separation width of the convex sections formed in the porous film surface of the cell culturing vessel. In addition, the three-dimensional culture skin sheet according to the invention is a three-dimensional culture skin sheet with low transepidermal water loss and a high barrier function compared to a conventional three-dimensional culture skin sheet. The barrier function is improved by the presence of the concavoconvexities in at least one portion of the base section 11 of the three-dimensional culture skin sheet. The base section 11 of the three-dimensional culture skin sheet is preferably closely bonded with the culture surface (the culture surface including the porous film and the convex sections). The barrier function is further improved if the base section 11 of the three-dimensional culture skin sheet is closely bonded with the culture surface. The base section 11 of the three-dimensional culture skin sheet is more preferably in a closely bonded state with the entire culture surface.

According to the invention, the convex sections provided in the surface of the porous film may be composed of biological substances, or composed of non-biological substances, or composed of a combination of biological substances and non-biological substances. Throughout the present specification, the term "non-biological substances" means substances excluding biological substances, i.e. biopolymers that constitute living bodies (nucleic acids, proteins, polysaccharides and their constituent elements (nucleotides, nucleosides, amino acids and sugars), and vitamins. The non-biological substance used to form the convex sections of the invention is preferably a biocompatible substance that does not affect culturing of the cells. According to the invention, the convex sections provided on the surface of the porous film may be further coated on their surfaces with a biological substance such as collagen, fibronectin, laminin, gelatin, vitronectin, polylysine (the D-form or L-form) or thrombospondin.

Conventional three-dimensional culture skin sheets have been fabricated by seeding epidermal cells onto a concavoconvex surface formed by pouring a gel containing proteins that are to form an extracellular matrix, into a mold having concavoconvexities (for example, U.S. Patent Application Publication No. 2011/0171180). In this method, however, since the entire culture surface is formed by a protein-containing gel, the proteins degrade during culturing, making it impossible for the cells to adhere and causing the three-dimensional culture skin sheet to contract. According to the invention, however, the convex sections are formed on a non-stretching porous film surface and the culture surface does not degrade even with culturing, thus making it possible to obtain a non-contracted three-dimensional culture skin sheet.

The cells to be used for the invention may be primary epidermal cells obtained by finely chopping biological tissue, and especially skin tissue, and treating it with a protease such as collagenase or trypsin, or they may be epidermal cells obtained by subculturing primary epidermal cells to cause their proliferation, or epidermal cells obtained by inducing differentiation from pluripotent stem cells such as ES cells, iPS cells or Muse cells, or they may be epidermal cells from an established line. Preferably, they are primary cultured epidermal cells obtained by harvesting from biological tissue and seeding, or subcultured epidermal cells obtained by further subculturing the primary cultured cells. If they are epidermal cells obtained by harvesting from biological tissue, they will be more likely to maintain the same properties as in the body, which is convenient when conducting tests for examining drug efficacy and side-effects, or for use in basic research.

According to the invention, the primary cultured epidermal cells are epidermal cells that have been harvested from biological tissue and then cultured only once in a cell culturing vessel and collected, and they may be referred to as "passage number: 0 (or first-generation)" epidermal cells. The non-subcultured epidermal cells that have become confluent or subconfluent may be subcultured by a method known to those skilled in the art, and further subjected to amplification culturing. Epidermal cells obtained by a single subculturing procedure are referred to as "passage number: 1" (or second-generation)" epidermal cells. The expression "passage number: 2, 3, 4 ... n (where n (integer) is the passage number) (n + 1 generations) may also be used, corresponding to the number of subcultures. Commercially available primary cultured cells (for example, frozen NHEK (NB), Catalog No.: KK-4009 by Kurabo Industries, Ltd.) include those provided as primary cultured cells from about passage number: 0 to passage number: 2 according to the invention, but the passage number in this case is the passage number according to the invention based on the passage number or number of generations listed in the documentation attached to the commercially available cells. A step of freezing the cells may also be included between each subculturing procedure.

Since most established somatic cell lines usually undergo changes in properties or variation in the proportion of constituent cells with repeated subculturing, they are altered into a cell population that is different from the cell population with a low number of passages. This is thought to result because the cells experience senescence due to shortening of telomeres with each cell division during repeated subculturing, and due to biological stress and/or physical stress resulting from confluency and/or trypsin treatment and the like. Usually, therefore, when using a tissue model comprising cultured cells, the cells are used with a lower number of passages.

However, since primary cultured cells are cells directly isolated from biological tissue and have a low number of passages, there are limits to the number of cells that can be supplied. The sizes and amounts of tissue that can be harvested are not only limited by ethical considerations, but variations also exist in the quality of the cells that can be obtained by donors. While primary cultured cells are commercially available, they are necessarily expensive due to supply volume issues. When it is necessary to construct a large three-dimensional culture skin sheet, therefore, it is necessary to obtain and use expensive cells, which raises a problem in terms of cost. For cheaper production, primary cultured cells that have been subcultured and proliferated may be used, but as mentioned above, the obtained cells are not necessarily limited to those exhibiting the same properties as primary cultured cells. Primary epidermal cells and kits for three-dimensionalizing the cells are commercially available and may be purchased for construction of a three-dimensional cultured skin model of epidermal cells (for example, keratinocyte three-dimensional culture starter kit by CELLnTEC), but when epidermal cells with a passage number of 2 or greater are used, the three-dimensionalizing nature spontaneously weakens, making it impossible to obtain a thick three-dimensional cultured skin model (also known as a three-dimensional culture skin sheet) (the controls of Examples 1 to 3 described below are examples of this).

According to an embodiment of the invention, if the three-dimensional culture skin sheet has concavoconvexities in its base section 11, a three-dimensional culture skin sheet having a thickness that has only been obtainable in the prior art with primary cultured cells (for example, passage number: 0 or 1) is constructed even when using epidermal cells with a passage number of 2 or greater after isolated culture from biological tissue. Even when primary cultured cells (for example, passage number: 0 or 1) are used, a thickened three-dimensional culture skin sheet according to the invention can be obtained. The passage number of the epidermal cells used after isolated culture from biological tissue may be 1, 2, 3, 4, 5, 6, 7, 8, 9, 10, or greater than 10, for example, with no limitation so long as the stratum spinosum, granular layer and/or stratum corneum of the three-dimensional culture skin sheet are thickened. According to an embodiment of the invention, the animal species of the primary epidermal cells used is not particularly restricted but is preferably a human. Also according to an embodiment of the invention, the primary epidermal cells used may be from a fetus, neonate, juvenile or adult, but they are preferably cells from a fetus, neonate or juvenile. When cells from a human juvenile are used, the cells may be of age younger than 20, such as 1 to 19, 1 to 10 or 1 to 5 years of age. Even when cells from a human adult are used, the cells used are preferably younger, such as 20 to 29, 30 to 39 or 40 to 49 years of age.

For one embodiment of the invention, the average thickness of the three-dimensional culture skin sheet may be 20 µm or greater, 21 µm or greater, 22 µm or greater, 23 µm or greater, 24 µm or greater, 25 µm or greater, 26 µm or greater, 27 µm or greater, 28 µm or greater, 29 µm or greater, 30 µm or greater, 35 µm or greater, 40 µm or greater, 45 µm or greater, 50 µm or greater, 55 µm or greater, 60 µm or greater, 65 µm or greater, 70 µm or greater, 75 µm or greater, 80 µm or greater, 85 µm or greater, 90 µm or greater, 95 µm or greater, 100 µm or greater, 105 µm or greater, 110 µm or greater, 115 µm or greater, 120 µm or greater, 125 µm or greater, 150 µm or greater, 175 µm or greater, 200 µm or greater, 225 µm or greater, 250 µm or greater, 275 µm or greater or 300 µm or greater. A more preferred average thickness is 80 µm or greater. The upper limit for the average thickness of the three-dimensional culture skin sheet is not particularly restricted since it will vary depending on the animal species, passage number and age of the epidermal cells.

### <Structure example (1) for cell culturing vessel for production of three-dimensional culture skin sheet>

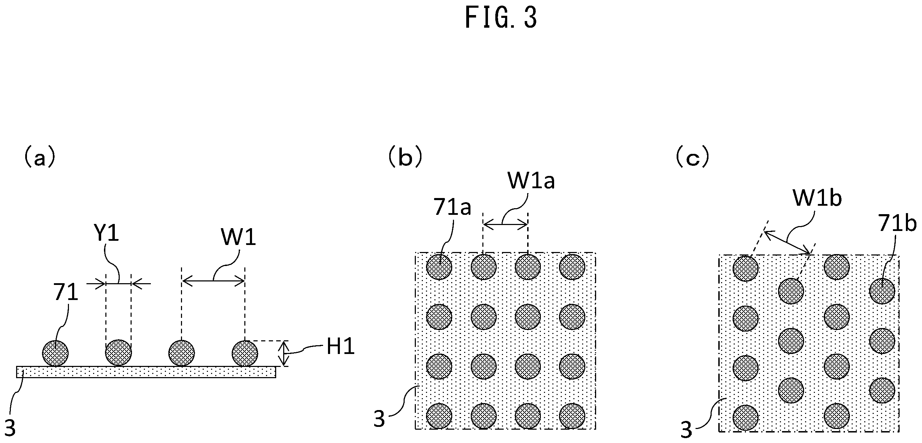

Fig. 1 mentioned above shows a conceptual drawing of a cell culturing vessel for creation of a three-dimensional culture skin sheet of the invention. Modified examples of the convex sections 7 in the cell culturing vessel 1 are shown in Fig. 3 to Fig. 5.

Fig. 3 shows an embodiment of a cell culturing vessel 1 of the invention. On the porous film 3, convex sections 71 are arranged for formation of concavoconvexities in the three-dimensional culture skin sheet of the invention. The convex sections 71 have bead shapes. By adjusting the height H1 of the convex sections it is possible to control the height H'1 of the irregularities of the three-dimensional culture skin sheet for the invention. As shown in Fig. 3(b), for example, the convex sections 71a may be arranged at the locations of square lattice points. By adjusting the separation width W1a between the convex sections, it is possible to control the separation width W'1 between the convex sections in the three-dimensional culture skin sheet of the invention. For example, as shown in Fig. 3(c), the convex sections 71b may be arranged at the locations of regular triangular lattice points. By adjusting the separation width W1b between the convex sections, it is possible to control the separation width W'1 between the convex sections in the three-dimensional culture skin sheet of the invention. Also, by adjusting the width Y1 of the convex sections, it is possible to control the width Y' of the recesses in the three-dimensional culture skin sheet of the invention.

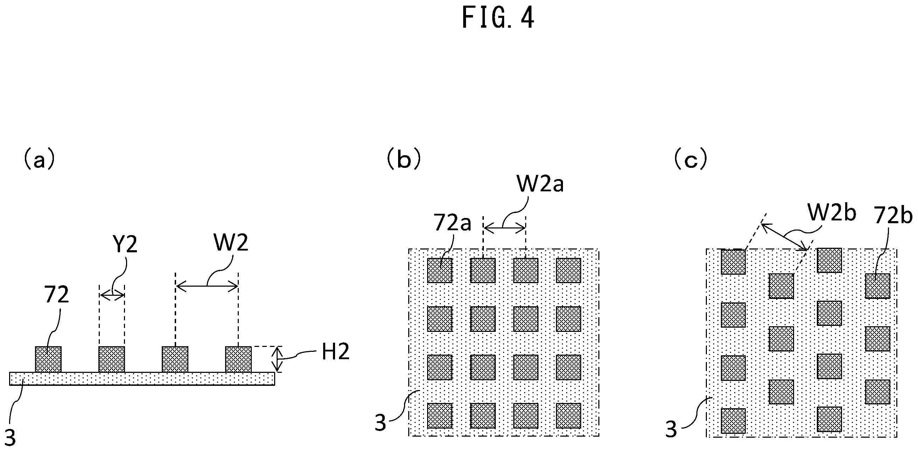

Fig. 4 shows an embodiment of a cell culturing vessel 1 of the invention. On the porous film 3, convex sections 72 are arranged for formation of concavoconvexities in the three-dimensional culture skin sheet of the invention. The convex sections 72 have cubic shapes, as types of rectangular columnar shapes. By adjusting the height H2 of the convex sections it is possible to control the height H'1 of the irregularities of the three-dimensional culture skin sheet for the invention. As shown in Fig. 4(b), for example, the convex sections 72a may be arranged at the locations of square lattice points. By adjusting the separation width W2a between the convex sections, it is possible to control the separation width W'1 between the convex sections in the three-dimensional culture skin sheet of the invention. As shown in Fig. 4(c), for example, the centers of gravity of the convex sections 72b may be arranged at the locations of regular triangular lattice points. By adjusting the separation width W2b between the convex sections, it is possible to control the separation width W'1 between the convex sections in the three-dimensional culture skin sheet of the invention. Also, by adjusting the width Y2 of the convex sections, it is possible to control the width Y' of the recesses in the three-dimensional culture skin sheet of the invention.

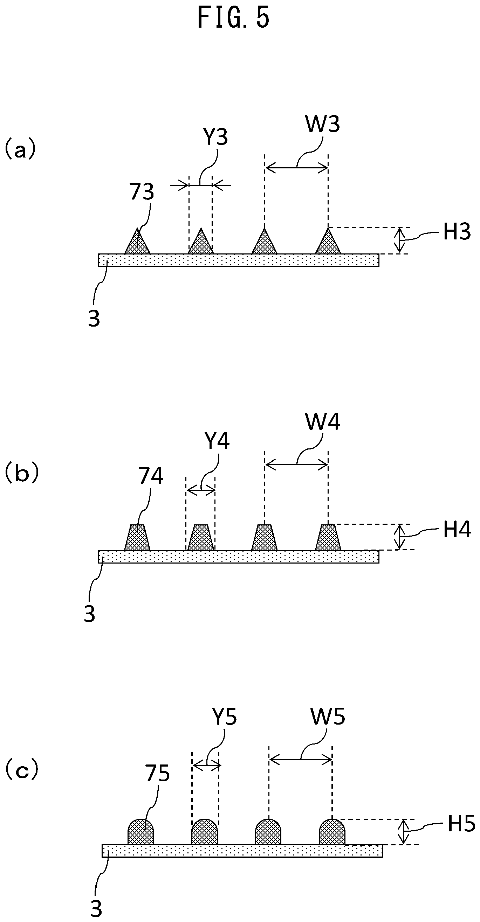

Fig. 5 is a cross-sectional view showing an embodiment of convex sections (73, 74, 75) provided on a cell culturing vessel 1 of the invention. Fig. 5(a), for example, shows convex sections 73 with pyramidal shapes (triangular pyramids, square pyramids or circular cones). Fig. 5(b), for example, shows convex sections 74 with frustum shapes. Fig. 5(c), for example, shows convex sections 75 with bell shapes. By adjusting the heights (H3, H4, H5) of the respective convex sections, it is possible to control the height H'1 of the irregularities of the three-dimensional culture skin sheet for the invention. By adjusting the separation widths (W3, W4, W5) between the convex sections, it is possible to control the separation width W'1 between the convex sections in the three-dimensional culture skin sheet of the invention. Also, by adjusting the widths (Y3, Y4, Y5) of the convex sections, it is possible to control the width Y' of the recesses in the three-dimensional culture skin sheet of the invention. The arrangement of the respective convex sections (73, 74, 75) on the plane may be the same arrangements as in Fig. 3 and Fig. 4, with no limitation thereto.

According to an embodiment of the invention, the shapes of the convex sections (7, 71, 71a, 71b, 72, 72a, 72b, 73, 74, 75) are not limited to those described above, and include approximately semi-spherical shapes and approximately cuboid shapes, for example. The material of the convex sections (7, 71, 71a, 71b, 72, 72a, 72b, 73, 74, 75) may be composed of biological substances, or composed of non-biological substances, or composed of a combination of biological substances and non-biological substances. Examples include polyesters or polyethylene terephthalate (PET), polycarbonate, polystyrene, zirconia, glass, insoluble collagen, silicon rubber and the like, or other materials so long as they allow cell culturing. The surfaces of the convex sections (7, 71, 71a, 71b, 72, 72a, 72b, 73, 74, 75) may also be subjected to plasma treatment by a publicly known method, in order to facilitate adhesion of the cells. The convex sections (7, 71, 71a, 71b, 72, 72a, 72b, 73, 74, 75) may be arranged at equal spacings on the porous film 3 as in the embodiments described above, or they may be arranged at non-uniform spacings, with an average lower limit for the spacings being 1 µm or greater, 5 µm or greater, 8 µm or greater, 10 µm or greater, 12 µm or greater or 15 µm or greater, for example. The average upper limit for the spacings between the convex sections (7, 71, 71a, 71b, 72, 72a, 72b, 73, 74, 75) may also be 300 µm or smaller, 250 µm or smaller, 200 µm or smaller, 180 µm or smaller, 170 µm or smaller, 150 µm or smaller, 125 µm or smaller or 100 µm or smaller, for example. According to the invention, the average spacing between the convex sections (7, 71, 71a, 71b, 72, 72a, 72b, 73, 74, 75) may be 1 µm to 300 µm, 5 µm to 250 µm, 8 µm to 200 µm, 10 µm to 180 µm, 12 µm to 100 µm, 25 µm to 80 µm or 30 µm to 75 µm, for example.

The average lower limit for the heights (H, HI, H2, H3, H4, H5) of the convex sections may be 1 µm or greater, 5 µm or greater, 10 µm or greater, 15 µm or greater, 20 µm or greater, 25 µm or greater or 30 µm or greater, for example. According to the invention, the average upper limit for the heights (H, H2, H3, H4, H5) of the convex sections may also be 300 µm or smaller, 250 µm or smaller, 200 µm or smaller, 150 µm or smaller, 100 µm or smaller, 80 µm or smaller or 75 µm or smaller, for example. According to the invention, the average of the heights (H, H2, H3, H4, H5) of the convex sections may be 1 µm to 300 µm, 5 µm to 250 µm, 10 µm to 200 µm, 15 µm to 150 µm, 20 µm to 100 µm, 25 µm to 80 µm or 30 µm to 75 µm, for example.

The average lower limit for the widths (Y, Y1, Y2, Y3, Y4, Y5) between the convex sections may be 1 µm or greater, 5 µm or greater, 10 µm or greater, 15 µm or greater, 20 µm or greater or 25 µm or greater, for example. According to the invention, the average upper limit for the widths of the convex sections (Y, Y1, Y2, Y3, Y4, Y5) may be 300 µm or smaller, 250 µm or smaller, 200 µm or smaller, 150 µm or smaller, 100 µm or smaller, 80 µm or smaller or 75 µm or smaller, for example. According to the invention, the average for the widths (Y, Y1, Y2, Y3, Y4, Y5) of the convex sections may be 1 µm to 300 µm, 5 µm to 250 µm, 10 µm to 200 µm, 15 µm to 150 µm, 20 µm to 100 µm, 25 µm to 80 µm or 30 µm to 75 µm, for example.

The convex sections (7, 71, 71a, 71b, 72, 72a, 72b, 73, 74, 75) may be anchored onto the porous film 3 in aggregates of several or several dozen or more. The method of anchoring the convex sections (7, 71, 71a, 71b, 72, 72a, 72b, 73, 74, 75) may be anchoring onto the porous film 3 in a covalently bonded manner by a publicly known method, or anchoring using an adhesive. The adhesive is preferably a substance that does not affect cell adhesion and has no toxicity for cells, and that is not degraded by the cells. Examples include poly-D-lysine and agarose, but any other adhesives may be used so long as they are substances that produce a similar effect and are able to anchor the convex sections (7, 71, 71a, 71b, 72, 72a, 72b, 73, 74, 75) onto the porous film 3. The convex sections (7, 71, 71a, 71b, 72, 72a, 72b, 73, 74, 75) may even be formed directly on the porous film 3, and for example, they may be formed by a method using 3D printer technology or semiconductor process technology (for example, a method combining a lithography process (including nanoimprinting or photoembossing) and dry etching (for example, reactive ion etching) or wet etching). Alternatively, the convex sections (7, 71, 71a, 71b, 72, 72a, 72b, 73, 74, 75) may be simply placed on the porous film 3.

### <Structure example (2) for cell culturing vessel for production of three-dimensional culture skin sheet>

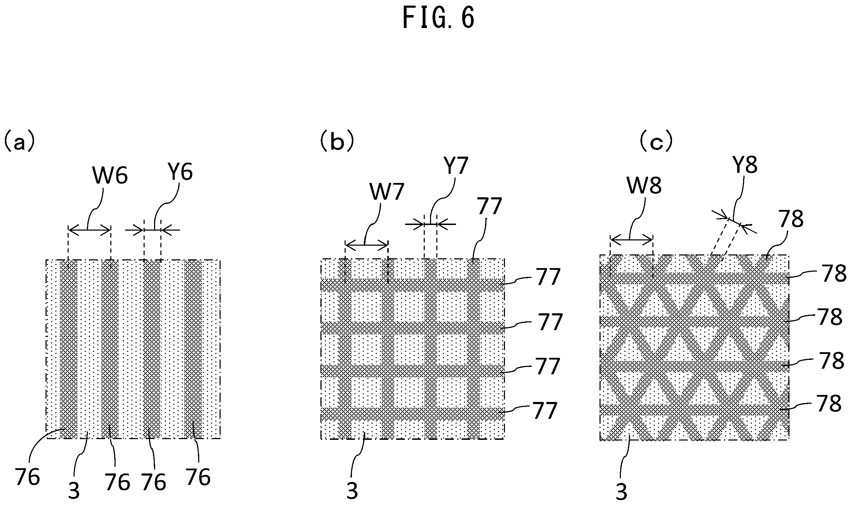

Fig. 1 mentioned above shows a conceptual drawing of a cell culturing vessel for creation of a three-dimensional culture skin sheet of the invention. Additional modified examples of the convex sections 7 in the cell culturing vessel 1 are shown in Figs. 6 and 7.

Fig. 6 shows an embodiment of a cell culturing vessel 1 of the invention. On the porous film 3, convex sections (76, 77, 78) are provided for formation of concavoconvexities in the three-dimensional culture skin sheet of the invention. For example, as shown in Fig. 6(a), the convex sections 76 may be arranged in an approximately parallel striped manner on the porous film 3. As shown in Fig. 6(b), for example, the convex sections 77 may be arranged in a lattice-like manner with equal spacings in the longitudinal and transverse directions on the porous film 3. As shown in Fig. 6(c), for example, the convex sections 78 may be arranged as a regular triangular lattice on the porous film 3. The convex sections (76, 77, 78) may be formed by fibers, for example. The convex sections (76, 77, 78) may be formed of single fibers, or they may be formed of bundles of multiple narrow fibers. Alternatively, the convex sections (76, 77, 78) may be formed as cuboid structures, or they may be constructed of approximately semicircular structures.

Fig. 7 shows an embodiment according to Fig. 6(b), which is a cell culturing vessel 1 comprising convex sections composed of alternately knitted convex sections 77a and convex sections 77b. Fig. 7(b) shows a cross-section on the line connecting Z and Z' in Fig. 7(a).

For this embodiment of the invention, the material of the convex sections (76, 77, 77a, 77b, 78) may be composed of biological substances, or composed of non-biological substances, or composed of a combination of biological substances and non-biological substances. Examples include polyesters or polyethylene terephthalate (PET), polycarbonate, polystyrene, glass, insoluble collagen, silicon rubber and the like, or other materials with no particular restrictions so long as the cells can adhere and culturing is possible. The average lower limit for the heights of the convex sections (76, 77, 77a, 77b, 78) may be 1 µm or greater, 5 µm or greater, 10 µm or greater, 15 µm or greater, 20 µm or greater, 25 µm or greater or 30 µm or greater, for example. According to the invention, the average upper limit for the heights of the convex sections may also be 300 µm or smaller, 250 µm or smaller, 200 µm or smaller, 150 µm or smaller, 100 µm or smaller, 80 µm or smaller or 75 µm or smaller, for example. According to the invention, the average of the heights of the convex sections (76, 77, 77a, 77b, 78) may be 1 µm to 300 µm, 5 µm to 250 µm, 10 µm to 200 µm, 15 µm to 150 µm, 20 µm to 100 µm, 25 µm to 80 µm or 30 µm to 75 µm, for example. For the embodiment shown in Fig. 7, the height H7a of the convex sections (77a, 77b) can be determined based on the uppermost sections (C, C') of the raised intersections where the convex sections 77a and convex sections 77b cross.

The convex sections (76, 77, 77a, 77b, 78) may be anchored at equal spacings or anchored at non-uniform spacings on the porous film 3, and the average spacing may have a lower limit of, for example, 1 µm or greater, 5 µm or greater, 8 µm or greater, 10 µm or greater, 12 µm or greater or 15 µm or greater. The upper limit for the average spacing between the convex sections (76, 77, 77a, 77b, 78) may be 300 µm or smaller, 250 µm or smaller, 200 µm or smaller, 180 µm or smaller, 170 µm or smaller, 150 µm or smaller, 125 µm or smaller or 100 µm or smaller, for example. According to the invention, the average spacing between the convex sections (76, 77, 77a, 77b, 78) may be 1 µm to 300 µm, 5 µm to 250 µm, 8 µm to 200 µm, 10 µm to 180 µm, 12 µm to 100 µm, 25 µm to 80 µm or 30 µm to 75 µm, for example. For the embodiment shown in Fig. 7, the separation width W7a between the convex sections can be determined based on the intersections (C, C') of the convex sections, where the convex sections 77a and convex sections 77b cross.

The widths (Y6, Y7, Y8) of the convex sections may be uniform or non-uniform. The average lower limit for the widths (Y6, Y7, Y8) of the convex sections may be 1 µm or greater, 5 µm or greater, 10 µm or greater, 15 µm or greater, 20 µm or greater or 25 µm or greater, for example. According to the invention, the average upper limit for the widths (Y6, Y7, Y8) of the convex sections may be 300 µm or smaller, 250 µm or smaller, 200 µm or smaller, 150 µm or smaller, 100 µm or smaller, 80 µm or smaller or 75 µm or smaller, for example. According to the invention, the average for the widths (Y6, Y7, Y8) of the convex sections may be 1 µm to 300 µm, 5 µm to 250 µm, 10 µm to 200 µm, 15 µm to 150 µm, 20 µm to 100 µm, 25 µm to 80 µm or 30 µm to 75 µm, for example.

The surfaces of the convex sections (76, 77, 77a, 77b, 78) may also be subjected to plasma treatment by a publicly known method, in order to facilitate adhesion of the cells. According to another embodiment, fibers that have been fragmented to arbitrary lengths may be used and anchored onto the porous film 3 to form the convex sections (76, 77, 77a, 77b, 78), or the convex sections (76, 77, 77a, 77b, 78) may be provided by contact bonding, or the convex sections (76, 77, 77a, 77b, 78) may be simply placed on the porous film 3. The method of anchoring the convex sections (76, 77, 77a, 77b, 78) onto the porous film 3 may be anchoring onto the porous film 3 in a covalently bonded manner by a publicly known method, or anchoring using an adhesive. The adhesive is preferably a substance that does not affect cell adhesion and has no toxicity for cells, and that is not degraded by the cells. Examples include poly-D-lysine, agarose, glucomannan and ultraviolet curing resins, but any other ones may be used so long as they are substances that produce a similar effect and are able to anchor the fibers onto the porous film. The convex sections (76, 77, 77a, 77b, 78) are preferably anchored onto the porous film 3 so that they do not detach in the cell culture. If a culture vessel 1 is used having the convex sections (76, 77, 77a, 77b, 78) and porous film 3 anchored, then it will be possible to obtain a three-dimensional culture skin sheet that not only is thickened but also has a high barrier function.

The material of the cell culturing vessel 1 used for the embodiments of the invention is not particularly restricted so long as it is a material commonly used for cell culturing vessels. Examples include glass, polystyrene, polypropylene and polycarbonate. The form of the cell culturing vessel used for the invention is also not particularly restricted, and it may be in the form of a dish, multi-well plate, flask or the like. Since the cell culturing vessel of the invention has a porous film on a portion of the cell culturing vessel, it is preferably in the form of a cell culture insert. A cell culture insert to be used for the invention is a cell culturing vessel comprising a film having porous holes that are impermeable to cells but permeable to culture solution. Culture solution may also be supplied from the opposite side of the culture surface of the porous film, i.e. the back side of the adhering surface of the adherent cells. The cell culture insert that may be used for the invention may be a commercially available one, or it may be a cell culture insert having a film provided with convex sections directly molded onto the culture surface of a porous film. When a commercially available one is used, the convex sections may be formed on the porous film surface by bonding beads or fibers, for example, or convex sections may be simply placed on the porous film 3 without bonding. When the convex sections are not to be bonded on the porous film 3, mesh-like convex sections of approximately the same diameter as the inside of the cell culture insert 2 (as in Fig. 7(a), for example) may be fitted into the cell culture insert 2 without bonding, for example. In this case, the convex sections may be provided together with or separately from the cell culture insert 2 as a portion of the culture vessel for preparation of the three-dimensional culture skin sheet, and they may be part of a kit to be assembled during culturing. The mean pore size of the porous film is not particularly restricted, and it may be 0.1 µm to 5.0 µm, preferably 0.2 µm to 4.0 µm and more preferably 0.3 to 3.5 µm, for example. The material used for the porous film may also be a polyester or polyethylene terephthalate (PET), or polycarbonate, polystyrene or the like, similar to porous films used in the prior art.

According to an embodiment of the invention, the culture surface may be coated to facilitate adhesion of the cells onto the cell culturing vessel that has convex sections on the porous film 3. Examples include collagen, fibronectin, laminin, gelatin, vitronectin, polylysine (the D-form or L-form) and thrombospondin.

### <Method for preparing three-dimensional culture skin sheet>

For an embodiment of the invention, the number of seedings of epidermal cells may be according to a publicly known method. For example, seeding of the epidermal cells may be in an amount of 0.01 × 10⁶ to 10.0 × 10⁶/cm², preferably 0.05 × 10⁶ to 5.0 × 10⁶/cm² and more preferably 0.1 × 10⁶ to 1.0 × 10⁶/cm². The culture medium (also referred to as "culture solution") to be used for culturing of the epidermal cells may be commonly used culture medium such as KG culture medium, EpilifeKG2 (Kurabo Industries, Ltd.), Humedia-KG2 (Kurabo Industries, Ltd.), assay culture medium (Toyobo), CnT-Prime or Epithelial culture medium (CELLnTEC), and culturing may be carried out at about 37°C for a period of 0 to 14 days. The culture medium used may alternatively be DMEM culture medium (Gibco), or a culture medium comprising a 1:1 mixture of 2-0-a-D-glucopyranosyl-L-ascorbic acid-containing KGM and DMEM. CnT-Prime, 3D barrier medium (CELLnTEC) may be used for three-dimensionalization (overlaying) of the epidermal cells. Other culture media may also be used.

For an embodiment of the invention, in order to culture the epidermal cells and achieve three-dimensionalization (overlaying) to promote keratinization, it is sufficient to seed the cells in a cell culturing vessel 1 containing a cell culture insert 2, and carry out growth culture. Specifically, the epidermal cells are suspended in medium and seeded onto the cell culture insert 2. In order to contact the culture medium with the outer side of the porous film 3 of the cell culture insert, culture medium is also added to the bottom well 4, and the cell culture insert 2 is immersed and cultured. As a result, the culture medium is supplied to both the top and bottom parts of the epidermal cells, and they are cultured.

The epidermal cells on the cell culture insert 2 are preferably cultured for about several days (about 1 to 6 days, and preferably about 2 to 4 days), until confluent or subconfluent. Next, to further promote three-dimensionalization (overlaying) of the cells, the culture medium inside and outside the cell culture insert is preferably exchanged with CnT-Prime, 3D barrier medium (CELLnTEC), for example. This will further promote three-dimensionalization of the epidermal cells. Upon exchange of the culture medium with CnT-Prime, 3D barrier medium (CELLnTEC) and culturing, preferably after 1 to 36 hours, preferably after 6 hours to 24 hours and more preferably after 12 hours to 18 hours, the culture medium inside the cell culture insert alone is removed and the top surfaces of the epidermal cells are exposed to a gas phase for culturing. This promotes three-dimensionalization and keratinization of the epidermal cells, yielding a thicker three-dimensional culture skin sheet.

Incidentally, the culturing temperature for each culturing step of the invention may be near the body temperature of the animal source, and specifically for human cells, it is preferably about 33°C to 38°C.

The three-dimensional culture skin sheet obtained by the invention in the manner described above can be used as an animal experiment substitute, such as a skin model. For example, it may be used in a method of evaluating reactivity of skin to chemical substances (for example, cosmetics, industrial products, household goods, drugs, external preparations for skin, and the like). In addition, since the three-dimensional culture skin sheet of the invention can provide thicker tissue compared to conventional three-dimensional culture skin sheets, it may be used as an effective skin model for basic dermatologic research as well. Furthermore, since the three-dimensional culture skin sheet obtained by the invention is thicker than conventional three-dimensional culture skin sheets, it has a higher barrier function from the outside and is also useful as a three-dimensional culture skin sheet for healing of burns and wounds.

According to one embodiment, the cell culturing vessel used for the invention may be a cell culturing vessel in which the convex sections and porous film are separable after completion of culturing. In this case, the three-dimensional culture skin sheet produced by the cell culturing vessel is separated from the porous film while the convex sections are still in contact with the base section. The base section of the three-dimensional culture skin sheet can thus be moved while maintaining the concavoconvex structure. In the cell culturing vessel used for the invention, the convex sections are preferably a biological substance including collagen, fibronectin, laminin, gelatin, vitronectin, polylysine (the D-form and L-form) or thrombospondin, for example, with insoluble collagen being more preferred.

### EXAMPLES

The present invention will now be explained in greater detail by examples, with the understanding that the invention is not limited in any way by the examples.

### <Preparation of cells>

The epidermal cells used for the invention were neonatal derived keratinocytes (hereunder, "keratinocytes"; product name Frozen NHEK (NB), Catalog No.: KK-4009, Kurabo Industries, Ltd.). Subculturing was carried out according to the instructions provided by the vendor.

### <Example 1>

(1) A dispersion of 10 mg of plastic beads (10 µm: M-310, 30 µm: M-503) by Matsumoto Yushi-Seiyaku Co., Ltd. in 1 mL of poly-D-lysine (1 mg/mL Millipore) was dropped in an amount of 10 µL onto 12-Well Millicell Hanging Cell Culture Inserts, 0.4 µm PET (product of Millipore, hereunder, "cell culture insert"), and dried at 70°C for 24 hours.
(2) CELLstart CTS (Gibco) was diluted 50-fold with DPBS (Gibco) and dropped in an amount of 86 µL onto one cell culture insert, and kept under conditions of 37°C for 2 hours.
(3) The CELLstart CTS was removed, 220,000 to 250,000 neonatal-derived keratinocytes with a passage number of 4 were dispersed in 500 µL of CnT-Prime, Epithelial culture medium (CELLnTEC) and dropped into the cell culture insert, 1 mL of the same culture medium (CnT-Prime, Epithelial culture medium) was dropped onto the exterior of the cell culture insert, and culturing was carried out in a CO₂ incubator at 37°C for 72 hours.
(4) The CnT-Prime, Epithelial culture medium inside and outside the cell culture insert was removed and exchanged with CnT-Prime, 3D barrier medium (CELLnTEC), and culturing was continued in the CO₂ incubator at 37°C for 16 hours.
(5) The culture medium inside and outside the cell culture insert was removed, the cell culture insert interior was exposed to air, and 500 µL of CnT-Prime, 3D barrier medium was placed outside the cell culture insert.
(6) Culturing was carried out for 7 days in a CO₂ incubator at 37°C while daily exchanging the 500 µL of CnT-Prime, 3D barrier medium outside the cell culture insert.
(7) The cultured epidermis of the inner film of each cell culture insert was cut out and fixed with a 4% paraformaldehyde-PBS solution, and a tissue section sample was prepared and stained with hematoxylin-eosin (HE) according to a publicly known method and observed under a microscope.

The results are shown in Fig. 8. With (a), wherein the culture surface of the cell culture insert was untreated, it was only possible to obtain a three-dimensional cultured epidermis with a thickness of about 10 µm, but when convex sections were provided in the cell culture insert surface by 10 µm PET beads (b) and 30 µm PET beads (c), thickening and stratum corneum formation of the three-dimensional culture cells were clearly observed. Incidentally, while it appears in Fig. 8(b) that the three-dimensional culture skin sheet has separated from the porous film of the cell culture insert, this separation occurred during preparation of the sample.

### <Example 2>

(1) A 12 mm-diameter polyester mesh was spread onto 12-Well Millicell Hanging Cell Culture inserts, 0.4 µm PET (Millipore, "cell culture insert"). The procedure thereafter was carried out in the same manner as (2) to (7) of Example 1.

The results are shown in Fig. 9. With (a), wherein the culture surface of the cell culture insert was untreated, it was only possible to obtain a three-dimensional cultured epidermis with a thickness of about 10 µm, but with the cell culture insert provided with convex sections (b), thickening and stratum corneum formation of the three-dimensional culture cells were clearly observed. In particular, the thickness of the stratum corneum was nearly twice that of the control (a). Incidentally, while Fig. 9(b) has partial hollowed sections, this was caused by handling during sample preparation.

### <Example 3>

An experiment was conducted to observe how changes in the width, separation width and height of the convex sections of the cell culturing vessel also caused changes in the thickness of the three-dimensional culture skin sheet.

Fig. 10 shows magnified views of polyester fibers used in the Examples for formation of concavoconvexities in a three-dimensional culture skin sheet of the invention.

(1) Fibers were spread onto 12-Well Millicell Hanging Cell Culture inserts, 0.4 µm PET (Millipore, "cell culture insert"). The procedure thereafter was carried out in the same manner as (2) to (7) of Example 1.

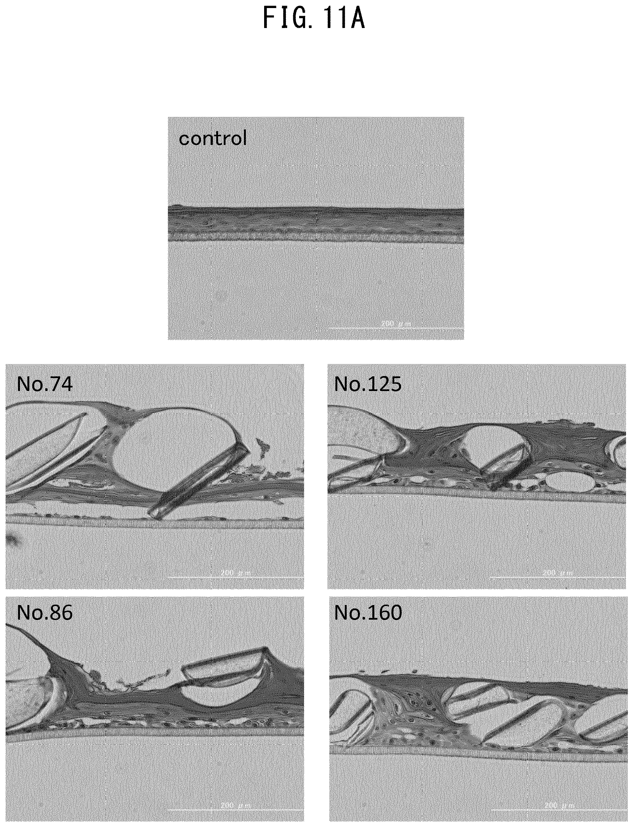

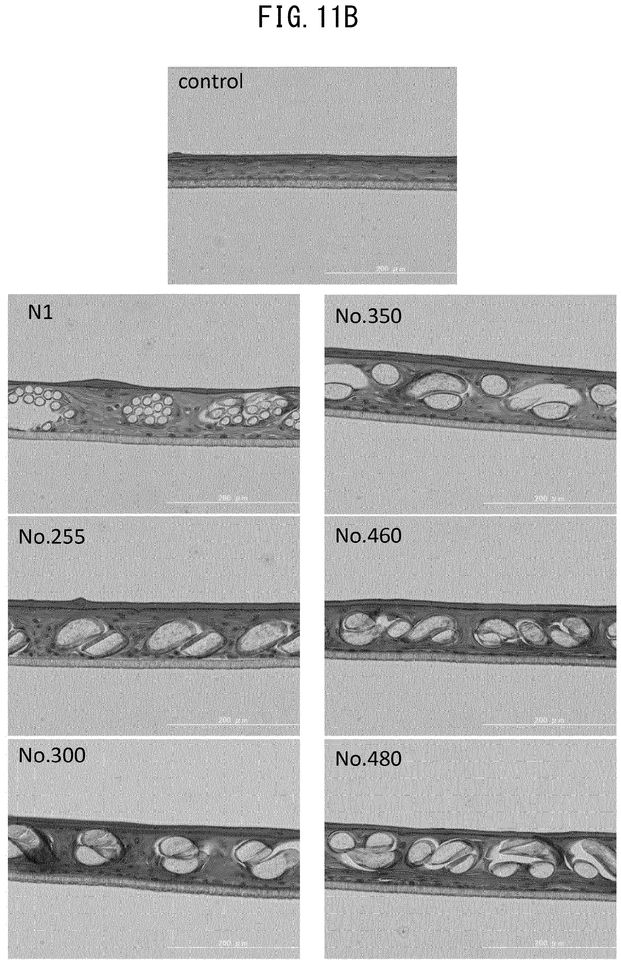

The characteristics of the fibers used for this example are shown in Table 1 below. Table 1 also shows the results that the convex sections formed using them had on formation of the three-dimensional culture skin sheet. The open area ratio was calculated based on the ratio of the area of the regions without mesh-like fibers, to the culture area. Fig. 11A and Fig. 11B show HE staining images of prepared three-dimensional culture skin sheet slices. Although sections with separation of the three-dimensional culture skin sheets and the porous films of the cell culture inserts, curling of the fibers and hollowing can be seen in Fig. 11A and Fig. 11B, these were all artificially created by handling during sample preparation.

**[Table 1]**

| Sample | Three-dimensional skin formation results(*) | Height (µm) | Separation (µm) | Fiber diameter (µm) | Fiber intersection area (µm × µm) | Open area ratio |
|---|---|---|---|---|---|---|
| No. 74 | F | 205 | 223 | 120 | 89.5 × 59.7 | 42% |
| No. 86 | F | 165 | 195 | 100 | 150 × 150 | 44% |
| No. 125 | F | 122 | 132 | 71 | 125 × 125 | 42% |
| No. 160 | F | 100 | 95 | 64 | 90 × 90 | 36% |
| N1 | VG | 55-65 | 71-164 | 53-93 | 80 × 80 | - |
| No. 200 | VG | 73 | 79 | 48 | 65 × 65 | 39% |
| No. 255 | VG | 60 | 60 | 40 | 55 × 55 | 36% |
| No. 300 | VG | 51 | 51 | 34 | 50 × 50 | 36% |
| No. 350 | G | 60 | 39 | 34 | 45 × 45 | 28% |
| No. 460 | G | 41 | 28 | 27 | 35 × 35 | 26% |
| No. 480 | G | 55 | 16 | 31 | 35 × 35 | 12% |

| | | | | | | |
|---|---|---|---|---|---|---|
| (*) P: Three-dimensional culture skin sheet was not thickened compared to the control, and lacked a denucleated horny structure. F: Three-dimensional culture skin sheet was thickened compared to the control, with a denucleated horny structure in portions. G: Three-dimensional culture skin sheet was thickened compared to the control, with a denucleated horny structure over almost the entirety. VG: Three-dimensional culture skin sheet was thickened compared to the control, with a denucleated horny structure over the entirety. | | | | | | |

### <Example 4>

(1) There were prepared a fabric of fibers No. 255 or No. 300 cut into the shape of a well and spread out onto 12-Well Millicell Hanging Cell Culture inserts, 0.4 µm PET (Millipore, "cell culture insert") ("No. 255 fabric" and "No. 300 fabric"), and cut No. 255 or No. 300 fibers bonded with an ultraviolet curing resin (Henkel, LOCTITE^{R} AA3554) ("No. 255 fabric-bonded" and "No. 300 fabric-bonded"). As a control, a well without convex sections provided by a fabric was used. Human skin (purchased from Biopredic International (France) via K.A.C. (Kyoto, Japan)) was used as another control. The procedure thereafter was carried out in the same manner as (2) to (6) of Example 1. The experiment was carried out in triplicate.

### <<Evaluation of barrier function>>

The barrier function was evaluated according to the following procedure.

An insert in which a three-dimensional epidermal model had been constructed was fitted into a silicon rubber vessel with culture solution in the bottom section, in a manner allowing moisture transpiration only from the epidermal model surface. The weight of the insert-fitted vessel was subsequently measured every other hour, and the reduction in weight was recorded as the moisture transpiration, calculating it to a numerical value per unit area. The method was based on the same principle as the method described in an article by Kumamoto (see Kumamoto J., Tsutsumi M., Goto M., Nagayama M., Denda M., Japanese Cedar (Cryptomeria japonica) pollen allergen induces elevation of intracellular calcium in human keratinocytes and impairs epidermal barrier function of human skin ex vivo. Arch. Dermatol. Res. 308:49-54, 2016).

The obtained results were statistically analyzed (ANOVA analysis of variance, followed by Scheffe multiple test analysis), and multiple test results with p < 0.05, compared to the three-dimensional culture skin sheet obtained by culturing with the film alone, were judged to have significant difference.

As a result, the three-dimensional culture skin sheet obtained with the cell culture inserts having the No. 255 fabric and No. 300 fabric bonded using an ultraviolet curing resin (No. 255 fabric-bonded and No. 300 fabric-bonded) had significantly lower transepidermal water loss compared to the three-dimensional culture skin sheet without concavoconvexities, demonstrating that a three-dimensional culture skin sheet with a high barrier function had been obtained (Fig. 12).

These results demonstrated that when a three-dimensional culture skin sheet is prepared with a cell culture surface having convex sections formed using a polyester mesh, a three-dimensional culture skin sheet is formed that is thicker and has a higher barrier function than by prior art methods.

### REFERENCE SIGNS LIST

1 Cell culturing vessel
2 Cell culture insert
3 Porous film
4 Bottom well
5 Culture medium
6 Culture surface magnified cross-section
7 Convex sections
8 Epidermal cells
9 Stratum corneum
10 Three-dimensional culture skin sheet
11 Base section (bold line)
71, 71a, 71b, 72, 72a, 72b, 73-77, 77a, 77b, 78 Convex sections
B1 Three-dimensional culture skin sheet recess
B2 Epidermal cell-free region bottom section
C, C' Uppermost sections of raised intersections
H, H1, H2 Heights of convex sections
H'1 Height of concavoconvexities of three-dimensional culture skin sheet
H'2 Height of epidermal cell-free region
H3-H5, H7a Heights of convex sections
T1 Raised section of three-dimensional culture skin sheet
T2 Top section of epidermal cell-free region
Va, Vb Epidermal cell-free regions
W, W1, W1a, W1b Separation widths of convex sections
W'1, W'2 Separation widths of convex sections of three-dimensional culture skin sheet
W2, W2a, W2b, W3-W8 Separation widths of convex sections
Y, Y1-Y8 Widths of convex sections
Y' Width of raised section of three-dimensional culture skin sheet

## Claims

1. A three-dimensional culture skin sheet comprising at least epidermal cells and having concavoconvexities in at least one portion of the base section.

2. The three-dimensional culture skin sheet according to claim 1, wherein the epidermal cells are keratinocytes.

3. The three-dimensional culture skin sheet according to claim 1 or 2, which further includes an epidermal cell-free region in at least one portion of the interior of the three-dimensional culture skin sheet.

4. The three-dimensional culture skin sheet according to any one of claims 1 to 3, wherein the average thickness of the three-dimensional culture skin sheet is 20 µm or greater.

5. The three-dimensional culture skin sheet according to any one of claims 1 to 4, wherein the height of the concavoconvexities is 1 µm to 300 µm on average.

6. The three-dimensional culture skin sheet according to any one of claims 1 to 4, wherein the height of the concavoconvexities is 1 µm to 90 µm on average.

7. The three-dimensional culture skin sheet according to any one of claims 1 to 6, wherein the separation width between the concavoconvexities is 1 µm to 300 µm on average.

8. The three-dimensional culture skin sheet according to any one of claims 1 to 6, wherein the separation width between the concavoconvexities is 1 µm to 100 µm on average.

9. The three-dimensional culture skin sheet according to any one of claims 1 to 8, which further comprises a porous film having convex sections that are in contact with the base section.

10. The three-dimensional culture skin sheet according to claim 9, wherein the convex sections are made of a non-biological substance.

11. A cell culturing vessel to be used for production of a three-dimensional culture skin sheet according to anyone of claims 1 to 10, comprising
a porous film provided on at least one portion of the culture surface and
convex sections arranged on the porous film and serving to form concavoconvexities on the base section of the three-dimensional culture skin sheet.

12. The cell culturing vessel according to claim 11, wherein the convex sections are made of a non-biological substance.

13. The cell culturing vessel according to claim 11 or 12, wherein the height of the convex sections is 1 µm to 300 µm on average.

14. The cell culturing vessel according to claim 11 or 12, wherein the height of the convex sections is 1 µm to 90 µm on average.

15. The cell culturing vessel according to any one of claims 11 to 14, wherein the separation width between adjacent convex sections is 1 µm to 300 µm on average.

16. The cell culturing vessel according to any one of claims 11 to 14, wherein the separation width between adjacent convex sections is 1 µm to 100 µm on average.

17. The cell culturing vessel according to any one of claims 11 to 16, wherein the convex sections comprise bead shapes, rectangular columnar shapes, pyramidal shapes, frustum shapes, bell shapes and/or lattice shapes.

18. A method for preparing a three-dimensional culture skin sheet, comprising:
(1) a step of seeding cells that include epidermal cells suspended in a culture medium, in a cell culturing vessel having a porous film provided on at least one portion of the culture surface and convex sections arranged on the porous film and serving to form concavoconvexities on the base section of the three-dimensional culture skin sheet, and
(2) a step of contacting the culture medium with the outer side of the porous film of the cell culturing vessel and culturing.

19. The method according to claim 18, further comprising:
(3) a step of removing the culture medium in the cell culturing vessel, exposing the epidermal cells to the air and culturing them.

20. The method according to claim 18 or 19, wherein the convex sections are made of a non-biological substance.

21. The method according to any one of claims 18 to 20, wherein the height of the convex sections is 1 µm to 300 µm on average.

22. The method according to any one of claims 18 to 20, wherein the height of the convex sections is 1 µm to 90 µm on average.

23. The method according to any one of claims 18 to 22, wherein the separation width between adjacent convex sections is 1 µm to 300 µm on average.

24. The method according to any one of claims 18 to 22, wherein the separation width between adjacent convex sections is 1 µm to 100 µm on average.

25. The method according to any one of claims 18 to 24, wherein the convex sections comprise bead shapes, rectangular columnar shapes, pyramidal shapes, frustum shapes, bell shapes and/or lattice shapes.

26. The method according to any one of claims 18 to 25, wherein the epidermal cells include epidermal cells with a passage number of 2 or greater after isolated from biological tissue and then cultured.

27. The method according to any one of claims 18 to 26, wherein the epidermal cells are keratinocytes.
